# EUROPEAN PATENT APPLICATION

(11) **EP 1 967 165 A1**
(43) Date of publication of application: **10.09.2008**
(21) Application number: 08250749.2
(22) Date of filing: 05.03.2008
(51) Int. Cl.: A61F 2/30

(54) **Optimized articular geometry**

(30) Priority: 09.03.2007 US 684028
(71) Applicant: Zimmer Technology, Inc., Warsaw, IN 46580 (US)
(72) Inventor: Hodorek, Robert A., Warsaw Indiana 46580 (US)
(74) Representative: Mays, Julie

(57) **Abstract**

Various embodiments of orthopaedic implants which optimize the articular geometries of the implants. An exemplary implant may include a plurality of protrusions which provide an articulating surface and which create paths for fluid movement relative to the implant.

## Description

### BACKGROUND

1. Field of the Disclosure.

The present disclosure relates to optimization of articular geometries of orthopaedic implants.

2. Description of the Related Art.

Many prosthetic joints include components having articulating surfaces which articulate with each other or with natural anatomical structures. For example, a prosthetic knee joint may include a femoral component, a tibial component, and a meniscal component, each of which may include articulating surfaces which articulate with each other. The prosthetic knee joint may be formed as a mobile bearing prosthetic knee joint, i.e., the meniscal component may rotate relative to and articulate with the tibial component. In the mobile bearing prosthetic knee joint, the tibial component and the meniscal component may have a flat-on-flat mating engagement, i.e., the surface of the tibial component in articulating engagement with the meniscal component is a flat surface and the surface of the meniscal component in articulating engagement with the tibial component is also a flat surface.

### SUMMARY

The present disclosure provides various embodiments of orthopaedic implants which optimize the articular geometries of the implants. An exemplary implant may include a plurality of protrusions which provide an articulating surface and which create paths for fluid movement relative to the implant.

In one form thereof, the present disclosure provides an orthopaedic implant including a body; and a plurality of macroscopic protrusions extending from the body, at least some of the protrusions defining articulating surface portions cooperating to define an articulating surface of the implant, wherein the articulating surface portions are substantially coplanar.

In another form thereof, the present disclosure provides an orthopaedic implant including a body; and optimization means associated with the body for providing enhanced lubrication to the implant.

In yet another form thereof, the present disclosure provides an orthopaedic implant including a body; and a plurality of substantially spherical protrusions extending from the body, at least some of the protrusions defining articulating surface portions cooperating to define an articulating surface of the implant.

In still another form thereof, the present disclosure provides an orthopaedic implant including a body; and a plurality of protrusions extending from the body, at least some of the protrusions defining articulating surface portions cooperating to define an articulating surface of the implant, the protrusions defining a path through which fluid can flow.

### BRIEF DESCRIPTION OF THE DRAWINGS

The above-mentioned and other features of the disclosure, and the manner of attaining them, will become more apparent and will be better understood by reference to the following description of embodiments of the disclosure taken in conjunction with the accompanying drawings, wherein:

Fig. 1 is a cross-sectional view of a prosthetic implant system including a first component and a second component according to an exemplary embodiment of the present disclosure;

Fig. 2 is a bottom view of one of the components of Fig. 1; and

Fig. 3 is a top view of one of the components of Fig. 1.

Corresponding reference characters indicate corresponding parts throughout the several views. The exemplifications set out herein illustrate embodiments of the disclosure and such exemplifications are not to be construed as limiting the scope of the invention in any manner.

### DETAILED DESCRIPTION

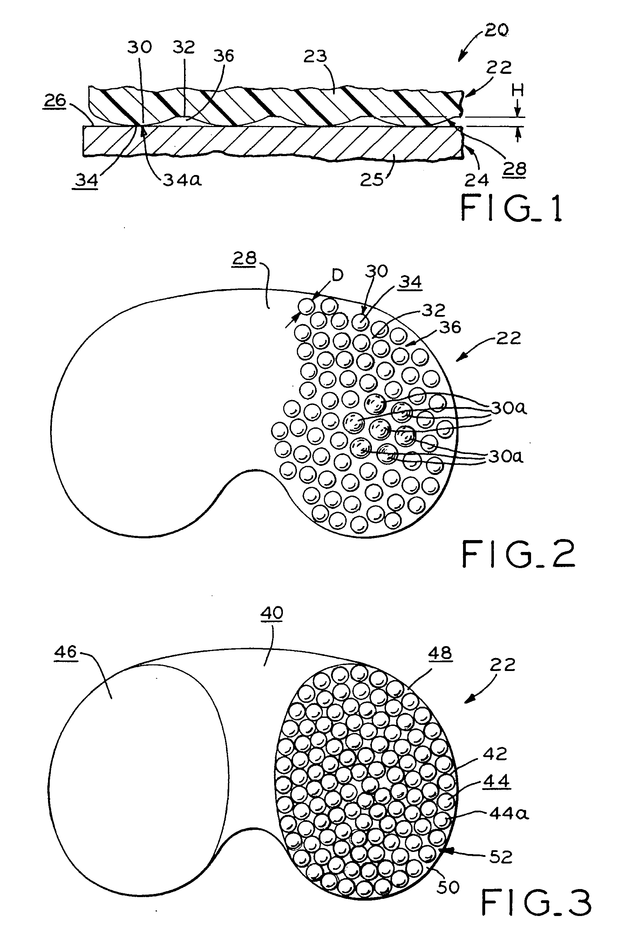

Referring now to Fig. 1, prosthetic implant system 20 may include first component 22 and second component 24. Second component 24 may include body 25 and mating surface 26 which may be substantially planar, e.g., surface 26 forms a substantially flat articulating surface with no macroscopic protrusions, i.e., surface 26 has only microscopic protrusions due to the inherent non-smooth microscopic nature of materials used to form second component 24. First component 22 may include body 23 and surface 28. Surface 28 may include a plurality of protrusions 30. Protrusions 30 each may include contact surface 34 for articulating engagement with surface 26 of second component 24. The raised portions of surface 28 including protrusions 30 define depressed areas 32. Each contact surface 34 may define apex 34a which defines an area, point, or region of protrusion 30 furthest from depressed areas 32. Depressed areas 32 may define paths or channels 36 between protrusions 30. Surface 28 may include a plurality of microscopic protrusions and depressions due to fundamental characteristics of the materials used to form first component 22, i.e., surface 28 is not perfectly smooth and may include a plurality of protrusions which are perceivable only with the aid of a microscope. Protrusions 30 are to be distinguished from such microscopic protrusions and extend a height H above a most depressed area of depressed areas 32, i.e., protrusions 30 are macroscopic protrusions which are large enough to be perceived or examined by the unaided eye. In exemplary embodiments, height H may be as small as approximately 0.1, 0.2, 0.4, 0.6, 0.8, 1.0, 1.5, 2.0, 2.5, or 3.0 mm or as large as approximately 6.0, 5.5, 5.0, 4.5, 4.0, or 3.5 mm. In one embodiment, channels 36 may define generally wedge-shaped openings, as illustrated in Fig. 1. In other embodiments, channels 36 may define a polygonal-shaped opening, a circular-shaped opening, or an elliptical-shaped opening. In an exemplary embodiment, channels 36 have a tapered cross-sectional area. In one embodiment, protrusions 30 may be formed to create discrete depressed areas 32 such as to define a plurality of discontinuous paths or channels 36, e.g., paths or channels 36 form pockets. In an exemplary embodiment, depressed areas 32 form a continuous network of paths or channels 36 to further enhance fluid movement between first component 22 and second component 24, as described below.

In an exemplary embodiment, surfaces 34 of each protrusion 30 together define an articulating surface of first component 22. As shown in Fig. 1, surfaces 34 are all substantially coplanar with respect to each other, i.e., surfaces 34 of each protrusion 30 are each substantially positioned in a single plane. For example, apexes 34a of surfaces 34 are all substantially coplanar.

Channels 36 provide passageways or paths for fluid to move between protrusions 30. Such fluid proximate the articulating surfaces of first component 22 and second component 24 enhances the amount of lubrication provided between first component 22 and second component 24 during articulation therebetween. The enhanced lubrication improves wear characteristics and properties of first component 22 and second component 24 by reducing the amount of wear imposed on each component. Furthermore, the geometry of protrusions 30 allows optimization of the amount of articulating contact area between first component 22 and second component 24 while maintaining channels 36 for flow of fluid lubricant therethrough. For example, the geometry of protrusions 30 provides a sufficient amount of surface contact between first component 22 and second component 24 while simultaneously providing enhanced lubrication therebetween. For example, contact surfaces 34 of protrusions 30 provide a substantial amount of contact between first component 22 and second component 24 such that articulation is not adversely affected during use of system 20. In exemplary embodiments, contact surfaces 34 may define an articulating surface area percentage of surface 28 (where depressed areas 32 define the remainder of surface 28) of as small as approximately 10%, 15%, 20%, 25%, 30%, 35%, 40%, 45%, or 50% or as large as approximately 95%, 90%, 85%, 80%, 75%, 70%, 65%, 60%, or 55%. The remainder of surface 28 defining depressed areas 32 provides the enhanced lubrication to the interface between first component 22 and second component 24. Moreover, the enhanced lubrication reduces friction between first component 22 and second component 24 while still allowing rotation and movement therebetween with lower forces imposed on first component 22 and second component 24. The reduced friction allows a more natural, i.e., non-prosthetic, movement within the joint, thereby allowing more normal rotations, movements, and kinematics of the components.

As shown in Fig. 2, depressed area 32 may form a continuous, depressed region which surrounds each protrusion 30. Each protrusion 30 may define a spherically-shaped dome. In other embodiments, each protrusion 30 may define other cross-sectional shapes, such as polygons and ellipses, with rounded contact surfaces 34. Protrusions 30 may vary in size on surface 28 and may have cross-sectional diameters D defined proximate depressed area 32. In exemplary embodiments, diameters D may be as small as approximately 0.2, 0.4, 0.6, 0.8, or 1.0 mm or as large as approximately 3.0, 2.5, 2.0, 1.8, 1.6, 1.4, or 1.2 mm. Also, protrusions 30a may be larger in cross-sectional size, i.e., protrusions 30a have a larger cross-sectional diameter than diameters D of protrusions 30. Protrusions 30a may be positioned on surface 28 in a location which incurs the highest load levels on first component 22 during articulation with second component 24. In an exemplary embodiment, multiple protrusions 30 and 30a carry the load imposed on first component 22 during articulation with second component 24. In an alternative embodiment, depressed area 32 may be formed as a plurality of discontinuous regions, such as a plurality of spherical depressions formed in surface 28.

As shown in Fig. 3, first component 22 may include second surface 40. In one embodiment, surface 40 is opposite surface 28 (Figs. 1 and 2). Second surface 40 may include a plurality of protrusions 42. Protrusions 42 are substantially similar to protrusions 30, 30a (Figs. 1 and 2), described above, and each may include contact surface 44 for articulating engagement with a third component (not shown) of prosthetic implant system 20. The raised portions of surface 40 including protrusions 42 define depressed areas 50. Each contact surface 44 may define apex 44a which defines an area, point, or region of protrusion 42 furthest from depressed areas 50. Depressed areas 50 may define paths or channels 52, similar to channels 36 (Figs. 1 and 2), described above, between protrusions 42. Surface 40 may include lateral/medial condyle surface 46 and lateral/medial condyle surface 48. Similar to protrusions 30, 30a (Figs. 1 and 2), described above, protrusions 42 may be formed in varying cross-sectional sizes. Although depicted in Fig. 3 positioned exclusively on surface 48 of first component 22, protrusions 42 may extend across the entire surface 40 and also extend from surface 46. Surfaces 44 of each protrusion 42 together define an articulating surface of first component 22.

Although shown in Fig. 3 as positioned on a condylar mating surface of first component 22 which may have a slight curvature, surfaces 44 of each protrusion 42 are all substantially coplanar with respect to each other, i.e., surfaces 44 are each substantially positioned in a single plane.

In an exemplary embodiment, system 20 is a prosthetic knee joint and first component 22 is formed as a meniscal or bearing component of the prosthetic knee joint, second component 24 is formed as a tibial component of the prosthetic knee joint, and the third component is formed as a femoral component of the prosthetic knee joint. Although described throughout the present disclosure as pertaining to a prosthetic knee joint, protrusions 30, 30a, 42 may be used on any orthopaedic implant which includes two components articulating against one another to reduce the wear and enhance the lubrication between the two components. Protrusions 30, 30a, 42 may also be used on any orthopaedic implant which includes a component which articulates against a natural anatomical structure to reduce the wear and enhance the lubrication between the component and the natural anatomical structure.

In one embodiment, protrusions 30, 30a, 42 may be formed by a net shape molding process. In another embodiment, protrusions 30, 30a, 42 may be formed by removing a portion of body 23 to define channels 36, 52. In yet another embodiment, protrusions 30, 30a, 42 may be formed by adding material to body 23 to create channels 36, 52.

In an alternative embodiment, protrusions 30 may be formed on second component 24 and first component 22 includes a substantially planar mating surface.

While this disclosure has been described as having exemplary designs, the present disclosure can be further modified within the spirit and scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims.

## Claims

1. An orthopaedic implant, comprising:
a body; and
a plurality of macroscopic protrusions extending from said body, at least some of said protrusions defining articulating surface portions cooperating to define an articulating surface of the implant, wherein said articulating surface portions are substantially coplanar.

2. The implant of Claim 1, wherein said protrusions define a plurality of paths therebetween.

3. The implant of Claim 2, wherein said paths form discrete pockets.

4. The implant of Claim 2, wherein said paths have a tapered cross-sectional area.

5. The implant of Claim 1, wherein at least some of said protrusions include an apex, each said apex defining one of said articulating surface portions.

6. The implant of Claim 5, wherein said apexes are substantially coplanar.

7. The implant of Claim 1, wherein said body includes a first side and a second side, said first side including said plurality of macroscopic protrusions extending therefrom, said second side including a second plurality of macroscopic protrusions extending therefrom, at least some of said second plurality of protrusions defining second articulating surface portions cooperating to define a second articulating surface of the implant.

8. The implant of Claim 1, wherein said protrusions comprise discrete protrusions.

9. The implant of Claim 1, wherein said protrusions vary in cross-sectional size.

10. The implant of Claim 1, wherein said protrusions are generally spherically shaped.

11. The implant of Claim 1, further comprising a second body, said second body including a substantially flat articulating surface.

12. An orthopaedic implant, comprising:
a body; and
optimization means associated with said body for providing enhanced lubrication to the implant.

13. The implant of Claim 12, wherein said optimization means comprises a plurality of macroscopic protrusions extending from said body, at least some of said protrusions defining articulating surface portions cooperating to define an articulating surface of the implant, wherein said articulating surface portions are substantially coplanar.

14. The implant of Claim 12, wherein said optimization means defines a plurality of lubrication paths.

15. The implant of Claim 14, wherein said paths have a tapered cross-sectional area.

16. An orthopaedic implant, comprising:
a body; and
a plurality of substantially spherical protrusions extending from said body, at least some of said protrusions defining articulating surface portions cooperating to define an articulating surface of the implant.

17. The implant of Claim 16, wherein said protrusions define a plurality of paths therebetween.

18. The implant of Claim 16, wherein said body includes a first side and a second side, said first side including said plurality of substantially spherical protrusions extending therefrom, said second side including a second plurality of substantially spherical protrusions extending therefrom, at least some of said second plurality of protrusions defining second articulating surface portions cooperating to define a second articulating surface of the implant.

19. The implant of Claim 16, wherein said protrusions vary in cross-sectional size.

20. An orthopaedic implant, comprising:
a body; and
a plurality of protrusions extending from said body, at least some of said protrusions defining articulating surface portions cooperating to define an articulating surface of the implant, said protrusions defining a path through which fluid can flow.

21. The implant of Claim 20, wherein said body includes a first side and a second side, said first side including said plurality of protrusions extending therefrom, said second side including a second plurality of protrusions extending therefrom, at least some of said second plurality of protrusions defining second articulating surface portions cooperating to define a second articulating surface of the implant, said second plurality of protrusions defining a path through which fluid can flow.

22. The implant of Claim 20, wherein said protrusions comprise discrete protrusions.

23. The implant of Claim 20, wherein said protrusions vary in cross-sectional size.

24. The implant of Claim 20, wherein said protrusions are generally spherically shaped.
